# EUROPEAN PATENT APPLICATION

(11) **EP 4 691 521 A1**
(43) Date of publication of application: **11.02.2026**
(21) Application number: 24192944.7
(22) Date of filing: 05.08.2024
(51) Int. Cl.: A61M 5/31, A61M 5/315

(54) **IMPROVED OPERATING ASSEMBLY FOR A SYRINGE**

(71) Applicant: Ypsomed AG, 3401 Burgdorf (CH)
(72) Inventor: Weiler, Rafael, 4053 Basel (CH)

(57) **Abstract**

The present invention is concerned with an operating assembly (1) and with a computer implemented method for a prefilled syringe (7) comprising: - a fixture (2) to accommodate the prefilled syringe (7), - a plunger rod (6) operatively coupled to the fixture (2 - a data acquisition unit (20) accommodated either by the fixture (2) or the plunger rod (6), - at least two interaction structures (11, 12), whereby one of the interaction structures is provided by the fixture (2) and the other by the plunger rod (6), wherein the interaction structures (11, 12) are mutually shaped and arranged in such a way a frictional force may act between them which occurs repeatedly during an ejection stroke wherein the acceleration and the speed of both the fixture (2) and the plunger rod (6) are modulated accordingly, wherein the data acquisition unit (20) captures a signal (27) evoked by said modulated speed and acceleration.

## Description

### FIELD OF THE INVENTION

The present invention relates to drug delivery devices or medicament administration devices for injecting, delivering, administering, infusing or dispensing substances and/or liquids such as insulin, hormone preparations or vaccines. It departs from an injection device comprising a reservoir and operating means configured for the administration of medicament.

### BACKGROUND OF THE INVENTION

A variety of diseases exist that require regular treatment by subcutaneous administration of a medicament, and several drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, where the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

Also known is the use of autoinjectors with syringes. Autoinjectors usually comprise a body for fixture a syringe as well as a drive mechanism in order to move the plunger of the syringe upon actuation of the autoinjector. The drive mechanism typically comprises a source of drive, such as a motor or strong spring for moving a transfer element, for example a rod, which acts on the plunger of the syringe.

For safety and hygiene reasons it is desirable that the needle does not protrude from a housing of the auto injector with the exception of the time when the needle is used for injection of a medicament. Thus, either the autoinjector moves the needle out of the housing for the injection and back into the housing after injection or the housing provides a needle guard which may be moved to unsheathe the needle for injection and which may be moved back to a needle covering position after the injection.

A plurality of medicament administration devices for injecting, delivering, administering is known as prefilled syringes PFS with or without needle safety devices NSD. NSD may accommodate a prefilled syringe PFS and are configured to ease the application thereof. For safety and hygiene reasons such NSD are configured so that the needle does not protrude from a housing of the NSD except for the time when the needle is used for injection of a medicament. By way of example, the PFS needle moves out of the housing for the injection and back into the housing after injection, or the housing provides or forms a needle guard which may be moved to unsheathe the needle for injection and which may be moved back to a needle covering position after the injection.

Prior art administration devices may include assemblies, devices or systems as disclosed in the following documents:
US2003144632 A1 The publication relates to an injection device including a tubular casing for a reservoir (ampoule or syringe) for a medicinal fluid, an injection needle, and a needle protecting sleeve which may be axially shifted on the casing between a protective position in which it surrounds the injection needle and a functional position in which it exposes the injection needle, wherein, in the protective position, the distal end of the needle protecting sleeve axially abuts at least one stopper and, in order to release the sleeve, the at least one stopper is radially shifted by applying a force to the casing.

WO18178888 A1 The publication is concerned with a simple, reliable manner of monitoring a process of expelling a variable dose of medicament from an injection device by way of analyzing mechanical feedback of the injection device with reduced requirements on signal data memory space and processing power. According to the invention, specific measurand and mechanical user feedback sensor configurations allow to produce sensor signal data from which individual dosage units of expelled drug are identifiable at moderate sampling rates. Preferred configurations include a force sensor adapted to measure an axial force component as applied to the injection device by a user pressing a proximal injection button, and a direction sensor adapted to measure an azimuth angle of an orientation of the injection device. The specific measurands, or process quantities observed, relate to feedback means of the injection device including a resilient element that produces a number of successive mechanical feedback events proportionate to a dose or quantity of drug expelled from a reservoir of the injection device.

WO14056635 A1 An injection device is disclosed, comprising: a) a casing which accommodates or forms a reservoir for a liquid drug; b) a plunger rod which can move relative to the casing in an axially forward direction to deliver the drug; c) a dosing means which can move in the forward direction relative to the casing to prime the reservoir and which can rotate relative to the casing for selecting a dose to be injected, wherein rotation of the dosing means relative to the casing is prevented until a priming operation for priming the reservoir is completed; d)wherein the dosing means comprises a dosing control means which is configured to enable rotation of the dosing means at the end of the priming stroke for selecting the dose and to prevent a rotation of the dosing means back into the position which the dosing means had at the end of the priming stroke relative to the casing).

WO05021071 A1 The publication relates to a system for administering an injectable product, comprising a hypodermic syringe, a drive unit and a housing accommodating the hypodermic syringe and the drive unit. The drive unit drives the hypodermic syringe in the longitudinal direction of the housing relative to the housing. Preferably, the hypodermic syringe is provided with a retraction device for retracting the hypodermic needle into the housing.

WO16033701 A1 The publication relates to an injection device comprising, amongst other things, a blocking device which can be switched from a blocking state into a release state. Said blocking device prevents, when in the blocking state, a tappet to be moved in the distal direction and allows, in the release state, the tappet to be moved by physical strength along a dispensing path in the distal direction. Said blocking device is switched from the blocking state into the release state by moving a needle stick guard from the starting position into the insertion position.

WO21237085 A1 The publication discloses a reporting injection device, including a barrel in fluid communication with a needle connected with a first end of the barrel, a piston including a plunger, the piston positioned within a second end of the barrel and the plunger having a fluid-tight interaction with an interior of the barrel, and a microprocessor in electronic communication with a switch and a wireless module, the microprocessor configured to send an administration data from the wireless module after triggering the switch.

WO20261270 A1 discloses an injection device and method for monitoring injection information relating to an injection. A syringe of the device includes a syringe chamber containing an injectant substance. A plunger of the device includes a plunger rod axially displaceable within the syringe chamber. At least one element of the device is configured to undergo a corresponding element motion linked to a distal displacement of the plunger rod. A sensor of an electronics unit of the device obtains detection readings relating to the corresponding element motion. An injection is performed by depressing the plunger rod in a distal direction, compelling a corresponding element motion detected by the sensor, and compelling the injectant substance through a distal end of the syringe chamber. The detection readings relating to the corresponding motion of the element is processed to determine injection information including at least the volume of injectant substance injected.

WO19099355 A1 The publication relates to an injection monitoring device including a syringe having a barrel configured to contain a medicament and a plunger configured to be displaced linearly into the interior of the barrel to dispense the medicament, a flange positioned between a proximal end and a distal end of the injection monitoring device and configured to be gripped during performance of the injection, and one or more force sensors positioned to detect data associated with an amount of force applied to the injection monitoring device during performance of an injection
WO18218167 A1 The publication relates to a syringe that includes a plunger having multiple sections of differing diameters or materials, and a barrel with a cavity into which the plunger is inserted. The barrel includes a first probe and a second probe opposite to one another such that an interior volume of the barrel is radially between the first and second probes. A microcontroller is configured to measure a capacitance between the first and second probes, with the measured capacitance having a first capacitance value when the first section of the plunger shaft is between the first and second probes and a second capacitance value different from the first capacitance value when a second section of the plunger shaft is between the first and second probes. The microcontroller is configured to determine that an injection has been completed when the second capacitance value is measured.

WO18218128 A1 discloses a syringe system, including a plunger, a microcontroller, a battery, and a coil. The syringe barrel having a proximal end, a distal end, and a cylindrical sidewall defining a longitudinal axis, the cylindrical sidewall extending longitudinally between the proximal and distal ends, the sidewall having an exterior surface and defining an internal volume, the plunger being positioned between the proximal and distal ends of the syringe barrel and being movable within the internal volume with respect to the syringe barrel in the longitudinal direction. The syringe barrel further includes a label disposed on the sidewall and having at least two conductive strips extending in a non-parallel direction with respect to the longitudinal axis and having unique lengths. The microcontroller is configured to determine a position of the plunger with respect to the syringe barrel by measuring a current induced in the coil by the at least two conductive strips.

WO18136901 A1 The publication discloses an injection system that can have a Syringe Dose and Position Apparatus (SDPA) mounted to a syringe. The SDPA can have one or more circuit boards. The SDPA can include one or more sensors for determining information about an injection procedure, such as the dose measurement, injection location, and the like. The SDPA can also include a power management board, which can be a separate board than a board mounted with the sensors. The syringe can also include a light source in the needle. Light emitted from the light source can be detected by light detectors inside a training apparatus configured to receive the injection. The syringe can have a power source for powering the sensors and the light source. The SDPA and the power source can be mounted to the syringe flange.

WO18125887 A2 The publication provides drug delivery systems, devices and methods of use for recording a drug dose completion signal in a data management system. Aspects of the invention include drug delivery systems comprising a syringe stopper rod that comprises a sensor component comprising a wireless transmitter module and an activation component configured to activate the sensor component when the syringe stopper rod has completed a delivery stroke, wherein the wireless transmitter module is configured to transmit a report comprising a drug dose completion signal when the sensor component is activated.

WO18111969 A1 discloses a smart plunger rod adapted to push a medication out of a syringe. The plunger rod comprises a shaft sized and dimensioned to act on a piston on the syringe and a finger actuated head portion containing at least two subunits of a wireless sensor. In a pre-activation configuration, the subunits are spaced apart from each other by a physical barrier and the wireless sensor is non-operational. In a post-activation configuration, the subunits are connected to each other and the sensor is operational to send a signal. A user activates the finger actuated head portion to reversibly move the physical barrier and moves the plunger rod from the pre-activation configuration to the post-activation configuration. The signal sent by the sensor comprises information relating to ejecting the medication out of the syringe, and is received by a remote receiver.

WO16140853 A1 Embodiments of the disclosure include a transponder for determining a characteristic of a dose delivery device. The transponder may include a first sensor configured to detect a first parameter of the dose delivery device based on movement of at least one of the dose delivery device or a medicament contained within the dose delivery device, wherein the first sensor is configured to generate a first signal indicative of the first parameter. The transponder may include a processor operably coupled to the first sensor and configured to process the first signal. The transponder may include an indicator unit operably coupled to the processor and configured to generate an output based on the first signal, wherein the output is perceivable by a user. The transponder may also include a battery configured to provide power to at least one of the first sensor, the processor, and the indicator unit.

WO16122974 A1 Described herein is a smart dose monitoring device. The dose monitoring device may include electronics such as motion sensors, orientation sensors, and timers related to monitoring the time of and amount of dose administered during an injection event.

WO14154870 A1 The disclosure relates to a finger grip arranged to be connected to a syringe barrel having a plunger and a plunger rod for driving said plunger. The finger grip comprises a body having a lower side with finger supporting surfaces for supporting fingers of a user during handling and an engagement member being moveable between an inactive position in which the engagement member is arranged not to engage with the plunger rod of the syringe barrel and an active position in which the engagement member is arranged to engage with a grooved surface on the plunger rod of the syringe such that feedback is given to a user as the plunger is moved relative to the finger grip. An activation member for moving the engagement member from said inactive position to said active position is built-in into the finger grip.

### DESCRIPTION OF THE INVENTION

It is an objective of the invention to further improve the safety in handling or use of operating assemblies or of administration devices including operating assemblies respectively. It is a further objective of the invention to provide simplified manufacturing and assembly and to reduce waste in production and after use of the devices.

These objectives are achieved by devices and methods according to the claims. Variants of embodiments of the invention are evident from the claims and this description.

The invention relates to an operating assembly for a prefilled syringe comprising:
- a fixture to fixedly accommodate the prefilled syringe, the prefilled syringe defining a central axis running through its distal needle end and its proximal filling end,
- a finger rest couplable to or integrally formed by the fixture,
- a bearing running with the central axis provided by the fixture, the bearing adapted for guiding axially e.g. coaxially
- a plunger rod operatively couplable or coupled to the fixture and axially movable in the bearing relative to the finger rest in an ejection stroke from a proximal initial position to a distal end position, the plunger rod comprising a button,
- a data acquisition unit accommodated either by the finger rest or the plunger rod, the data acquisition unit comprising: a processor, a memory, a transceiver and a sensor, the sensor comprising at least one of an acceleration detector, a microphone or a strain gauge
- at least two interaction structures, where one of the interaction structure is provided by the fixture and the other by the plunger rod, where the interaction structures are mutually or complementary shaped and arranged in such a way that a frictional force may act between them which occurs and /or varies repeatedly e.g. periodically during or along an ejection stroke where the relative and/or absolute acceleration and the relative and/or absolute speed of both the fixture and the plunger rod and/or evoked acoustical sound and/or structure borne sound are modulated accordingly, where the data acquisition unit captures a mechanical and/or acoustical signal evoked by said frictional forces or the interactions structures respectively e.g. by transforming said signal into a corresponding electrical signal and/or a digital representation. Additionally, or alternatively to a microphone, a tension detector, a pressure detector or a strain gauge, the sensor may include an accelerometer to transform said acceleration or speed changes into a corresponding electrical signal and/or digital representation.

The intended use and save and compliant administration of modern medicaments needs conformant devices and methods where safe usage is enabled and eased with the improvements according to the invention. Information gathered with devices and methods according to the invention is crucial to systems providing user support or monitoring administration/treatment/injection adherence.

Besides that, devices according to the invention may be assembled and are applicable with a minimal number of parts and different materials to reduce the handling and tooling effort in production for cost sensitive and modular platform variants or environmentally friendly applications and safe disassembly, disposal, or reuse.

In the following partial elements and/or features according to the invention are defined and described exemplarily in a possible context and/or use.

The fixture with the finger rest is part of the disposable or reusable operating assembly. To prepare the administration device for an administration the final assembly provider or the user may attach or connect the reservoir unit or the syringe unit to an operating assembly. The operating assembly may include drive means adapted to dispense the liquid drug from the reservoir unit once the reservoir unit is attached to the operating assembly.

Such an administration device comprising an operating assembly may be a manual or an automatic administration device e.g. an automatic injection device or an autoinjector or an autopen. Automatic devices typically include automatic drive means such as an elastic element (for example a pretensioned spring) or an electric motor to drive a dispensing member to dispense the liquid drug form the reservoir e.g. from a prefilled syringe or from a prefilled cartridge both providing a barrel containing the medicament and a moveable stopper as a dispensing member. Manual injection devices include a manual drive such as a dispensing button or a plunger rod that must be pressed by the user in order to move the dispensing member e.g. the stopper in the barrel in dispensing direction. The fixture and the plunger rod may be separate plastic parts. The term "separate" means that before assembly the parts can be handled independently from each other and may be produced separately and independently from other parts. This does not exclude that the parts may be connected upon assembly of the device. The parts may be connected to each other, for example, by a snap-fit and/or a thread and/or linear guiding structures.

The data acquisition unit may be a separate module adapted to be easily and safely assembled or integrated or accommodated in one of the plunger rod or the fixture. The data acquisition unit comes as an autonomous module with processing, storing, communication capabilities and with its own on-board energy supply. The data acquisition unit may fulfil the requirements of an Internet of Things IoT node. It may be further equipped with input devices for physical phenomenon like acoustic or structure borne waves or strain or tensile force and compressive force as well as for acceleration and temperature. Further it may feature local output channels like a single or multi-LED display or a sound generator.

For the computer implemented methods a copy of a computer program stored on a computer-readable medium in the form of an application program is installed on the data acquisition unit of the operating assembly or the injection device respectively. The computer program causes, when being executed by a processor of the data acquisition unit for monitoring an injection process, the processor to execute some or all the disclosed method steps. The computer-readable medium may be a RAM (Random Access Memory), a ROM (Read Only Memory), an EPROM (Erasable Programmable Read Only Memory), or a data communication network, e.g. the Internet, which allows downloading program code.

The features of the methods of the data acquisition unit and the controller as described herein may be performed by way of hardware components, firmware, and/or a computing device having processing means controlled by appropriate software. For instance, the controller can include any known general-purpose processor or integrated circuit such as a central processing unit (CPU), microprocessor, system on chip (SoC), field programmable gate array (FPGA), Application Specific Integrated Circuit (ASIC), or other suitable programmable processing or computing device or circuit as desired. The processor can be programmed or configured to include and perform features of the exemplary embodiments of the present disclosure such as, a method carried out by the data acquisition unit of the operating assembly or the injection device respectively according to the invention. The features can be performed through program or software code encoded or recorded on the processor or stored in a non-volatile memory accessible to the processor, such as Read-Only Memory (ROM), erasable programmable read-only memory (FLASH, EPROM), or other suitable memory or circuit as desired. In another exemplary embodiment, the program or software code can be provided in a computer program product having a non-transitory computer readable recording medium such as a hard disk drive, optical disk drive, solid state drive, a USB drive or other suitable memory device or circuit or network as desired, the program or software code being transferable or downloadable to the processor for execution when the non-transitory computer readable medium is placed in communicable contact with the processor.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "injection system" or "injector" refers to an administration device that is removed from the injection site after each medication event or drug delivery process, whereas the term "infusion system" refers to a device with a cannula or needle that remains in the skin of the patient for a prolonged period of time, for example, several hours.

In the claims, the word "comprising" does not exclude other elements or steps, and the indefinite article "a" or "an" does not exclude a plurality. For example, "an arm" does not exclude the fact that there may be two arms that functionally or structurally fulfill the purpose of "an arm". The mere fact that certain elements or steps are recited in distinct claims shall not preclude the existence of further meaningful combinations of these elements or steps.

The term "distal" is meant to refer to the direction or the end of the drug delivery device carrying an injection needle or an injection cannula, whereas the term "proximal" is meant to refer to the opposite direction or end pointing away from the needle or cannula.

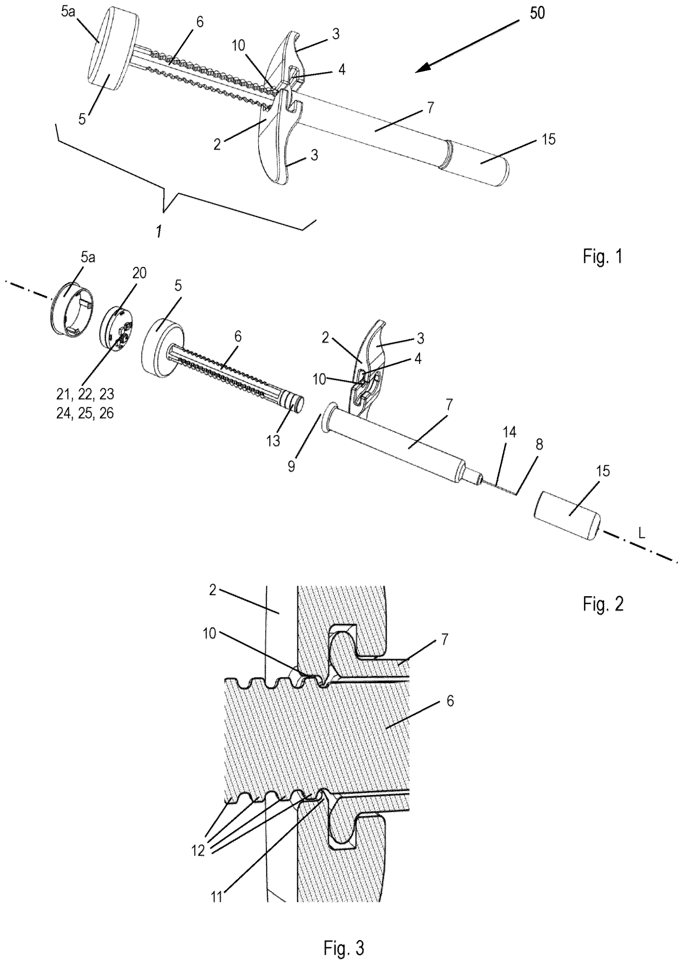

The term "proximal" refers to the left-hand side in the figures 1, 2, 4, 5. The term "distal" refers to the opposite side or rear end of the device and is on the right-hand side in the figures. In the following the structural features and parts of the operating assembly and the administration device 50 according to the invention will be descripted exemplarily. Subsequently, the function and use of the embodiments will be explained.

The operating assembly can be built in a variant embodiment of the invention, where the interaction structure provided by the plunger rod comprises a plurality of radially directed protrusions and/or indentations formed along a surface section of the plunger rod. The interaction structure provided by the plunger rod may be moulded in one piece with the plunger rod. These arrangements allow for optimized coupling of the signal to the data acquisition unit.

The operating assembly can be built in a variant embodiment of the invention, where the interaction structure provided by the fixture comprises at least one of a protrusion and/or indentation provided by the bearing or formed along a surface section of the bearing. The interaction structure provided by the fixture may be moulded in one piece with the fixture. These arrangements allow for optimized coupling of the signal to the data acquisition unit.

The operating assembly can be built in a variant embodiment of the invention, where during or along the ejection stroke a mutual deformation and/or deflection of the interaction structures takes place. In a further variant embodiment of the invention the interaction structures may be configured as a ratchet e.g. having a claw on the fixture and a toothed rack on the plunger rod to allow an injection stroke movement in a distal direction but not a movement in the reverse direction. These arrangements allow for ejection safety without aspiration and blocks unintended reuse.

This mutual deformation and /or deflection interacts with friction forces influencing or modulating a thrust causing the ejection stroke and therefore acceleration and speed of the involved parts e.g. the fixture, the plunger rod. This variations in acceleration and speed can easily be sensed by detectors with improved precision. Additionally, and effecting a synergistic effect, the interaction structures alternately undergo static and dynamic friction. This modulation results in an improved control of the manual thrust applied on the button and/or the plunger rod to move the stopper in the barrel of the prefilled syringe and therefore in better precision of the ejection of the prefilled syringe e.g. a full, partial or stepwise ejection of a dedicated dose of medicament. Additionally, the number of signals counted can further aid the detection of injection completeness.

The operating assembly can be built in a variant embodiment of the invention, where the plunger rod is moved axially through the bearing and into the prefilled syringe by a manual thrust acting on a proximal end plate of the button on the plunger rod and the finger rest of the fixture.

The operating assembly can be built in a variant embodiment of the invention, where the distal end plate of the button and the proximal side of the fixture are provided with interlocking hooks. The interlocking hooks are configured to irreversibly lock the plunger rod in its distal end position to the fixture after the plunger rod has been moved to the end of the ejection stroke. Harmful reuse of the operating assembly is prevented. The sensor of the data acquisition unit may be adapted to detect the distinctive signal evoked by the "snap-in" of the interlocking hooks at the end of the injection stroke.

Together with the signal evoked by the interaction structure the event recognition and discrimination by the data acquisition unit may substantially be enhanced.

The operating assembly can be built in a variant embodiment of the invention, where the sensor of the data acquisition unit further comprises a pressure or force detector capable of capturing a force acting on the button from the outside.

This additional detector allows to monitor the course of manual thrust applied on the plunger rod along the ejection stroke, where a distinct increase of the measured force is detectable if the stopper in the prefilled syringe reaches the end of the barrel, or the plunger rod hits a fixed or variable axial stop structure in the fixture. The stop structure may be adapted to form a latch for example said interlocking hooks. This distinct or transient increase and/or decrease of the measured force may be interpreted as an "end of injection" or "injection performed" event. Such information is crucial to systems providing user support or monitoring user adherence.

The operating assembly can be built in a variant embodiment of the invention, where the data acquisition unit further includes a display, for example a LED providing status information e.g. "device ready", "system paired", "injection performed". The button and/or the lid may be formed from transparent material or may exhibit a window to enable the display.

As well an injection device including an operating assembly is a further embodiment according to the invention, the injection device further including:
- a prefilled syringe having a stopper and a staked needle, the staked needle covered by a removable needle shield, the prefilled syringe accommodated in the operating assembly, for example attached to the fixture, where the flange of the prefilled syringe is fastened by a clamping groove in the fixture.

A further embodiment according to the invention is represented by an injection device comprising an operating assembly, the injection device further including:
- a safety sleeve telescopically movable with respect to the prefilled syringe capable to expose the needle for injection. The safety sleeve is urged by a safety spring in distal direction. The safety spring is tensioned as the needle is exposed.

The safety spring and/or the safety sleeve are moveably accommodated in the annular gap between the barrel of the syringe and a housing sleeve which coaxially surrounds the barrel of the syringe.

The safety sleeve improves stabbing safety and mitigates contamination hazard.

The injection device can be built in a variant embodiment of the invention, where the injection device further includes a cap, operatively coupled to the needle shield and adapted to remove the needle shield from the prefilled syringe together with the cap.

The cap is removably coupled with the fixture and/or with the syringe by the housing sleeve.

Ease of preparing the device for use is improved.

A computer-implemented method carried out by the data acquisition unit of the operating assembly or the injection device respectively according to the invention, the method including the steps of:
- predefining, in the memory, data patterns characteristic for or relating to signals evoked by at least one of acceleration, structure borne sound or strain taking place in one or more of the plunger rod or the fixture or other parts during intended use of the administration device
- capturing, by the sensor, the signal evoked by at least one of acceleration, structure borne sound or strain taking place with or inside one or more of the plunger rod, the fixture or further involved parts or usage actions
- receiving, by the processor, from the sensor the electrical representation of the signal rendering in the time domain at least one of said acceleration, acoustical or structure borne sound or strain
- storing, in the memory, the digital representation of said signal e.g. in the time domain
- transforming and storing, by the processor, in the memory, the digital representation of the signal into other domains, for example the frequency domain
- obtaining, by the processor, by comparing or correlating the digital representation of signal and the predefined patterns in memory, metrics assigned to the relative movement of the plunger rod or the button and the finger rest or the fixture, for example a relative or absolute position and/or the speed of the plunger rod with respect to the other parts e.g. the fixture, the prefilled syringe or other parts.

The method further including at least one of the steps of:
- converting, by the processor, the metrics into an amount of medication expelled
- obtaining, by the processor, by comparing or correlating the digital representation of the signal and the predefined patterns in memory, device status information

In variant embodiments of the invention said data acquisition unit or methods are adapted to sense and process signals evoked by removal of the cap and/or of
the needle shield or by movements of the safety sleeve.

The method further including the steps of:
- pairing, by the processor and the transceiver, the device with a remote gateway device, for example a mobile device, an internet of things IoT node, a smartphone and/or a smartwatch, the gateway device connectable or connected to a host or datacenter via a network
- transmitting, by the transceiver, metrics and status to the remote gateway device
- receiving, by the transceiver, data to be stored in memory or to be shown on the display

The method according to the invention, where the predefined data patterns are obtained by training a neural network or by machine learning or by the use of artificial intelligence.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a perspective view of a first embodiment of an operating assembly and an injection device respectively according to the invention;
- Fig. 2: depicts an exploded perspective view of the operating assembly and the injection device respectively from figure 1;
- Fig. 3: depicts a sectional view of an axial section of an operating assembly and an injection device respectively from figure 1;
- Fig. 4: depicts a perspective view of a second embodiment of an operating assembly and an injection device respectively according to the invention;
- Fig. 5: depicts an exploded perspective view of the operating assembly and the injection device respectively from figure 4;
- Fig. 6: depicts a sectional view of an axial section of an operating assembly and an injection device respectively from figure 4;
- Fig. 7: depicts a perspective view of a fixture according to the invention;
- Fig. 8: depicts a perspective view of a button according to the invention;
- Fig. 9: depicts a sectional view of the proximal section of an operating assembly and an injection device respectively;
- Fig. 10: depicts a flowchart of a computer implemented method for an embodiment according to the invention;
The reference symbols used in the drawings, and their primary meanings, are listed in summary form in the list of designations. In principle for the variant embodiments, identical parts or equivalent functional units are provided with the same reference symbols in the figures.

### DETAILED DESCRIPTION OF VARIANT EMBODIMENTS

The term "proximal" refers to the left-hand side in the figures 1, 2, 3, 4, 5. The term "distal" refers to the opposite side or rear end of the device and is on the right-hand side in said figures. In the following the structural and/or functional features and the parts of the operating assembly 1 and the administration device 50 according to the invention will be described exemplarily. Subsequently, the function and use of the administration device 50 will be explained.

Figure 1 depicts a perspective view of a first embodiment of an operating assembly 1 and an injection device 50 respectively according to the invention. The operating assembly 1 includes a fixture 2, a plunger rod 6 and a button 5. The injection device 50 includes said operating assembly 1 where a syringe 7 is fastened in the fixture 2 by the clamping groove 4. The distal end of the syringe 7 is covered by a needle shield 15. The fixture 2 forms a finger rest 3 on the distal side of a lateral protrusion. The fixture 2 may operatively guide the plunger rod 6 in the bearing 10 for an axial movement e.g. an ejection stroke. At the proximal end of the plunger rod 6 a distinct button 5 is formed or attached. A lid 5a may close the interior of the button 5 and/or may form a thumb rest at the proximal end of the operating assembly 1.

Figure 2 depicts an exploded perspective view of the injection device 50 respectively from figure 1. From left to right along the central axis L the following parts are shown: The lid 5a, a data acquisition unit 20accommodated in the button 5 including a processor 21, a memory 22, a transceiver 23, a sensor 24, a battery 25 and a display 26. The display 26 e.g. a LED may be visible through a transparent sidewall of the button 5 or through the lid 5a. The distal end of the plunger rod 6 carries a stopper 13. A syringe 7 with a staked needle 14. The prefilled syringe 7 features a proximal filling end 9 and a distal needle end 8. The fixture 2 with the finger rest 3 features a lateral opening or clamping groove 4 and a central or coaxial opening or bearing 10. The bearing 10 is adapted to guide the plunger rod axially along the central axis L. The clamping groove 4 is adapted to take in and clamp the flange and a section of the barrel of the syringe 7. The releasable needle shield 15 is adapted to sealingly cover the staked needle 14.

Figure 3 depicts a sectional view of an axial section of the operating assembly 1 and of the injection device 50 respectively from figure 1. The plunger rod 6 and the fixture 2 with the clamped syringe 7 is shown. The bearing 10 exhibits interaction structures 11,12 where a first interaction structure 11 may be formed by the fixture 2 by a radially inwardly directed protrusion and a second interaction structure 12 may be formed by the plunger rod 6 as radially outwardly directed and axially lined up protrusions. As the plunger rod 6 is guided and axially moved in the bearing 10 of the fixture 2 variable frictional force and/or plastic deformation occurs between and on the interaction structures 11, 12. Such acoustical and/or structure-borne sound is evoked and the speed of the plunger rod 6 is modulated periodically by the variation of the frictional force as the plunger rod with its interaction structure 12 is moved by an external thrust in e.g. an injection or ejection stroke.

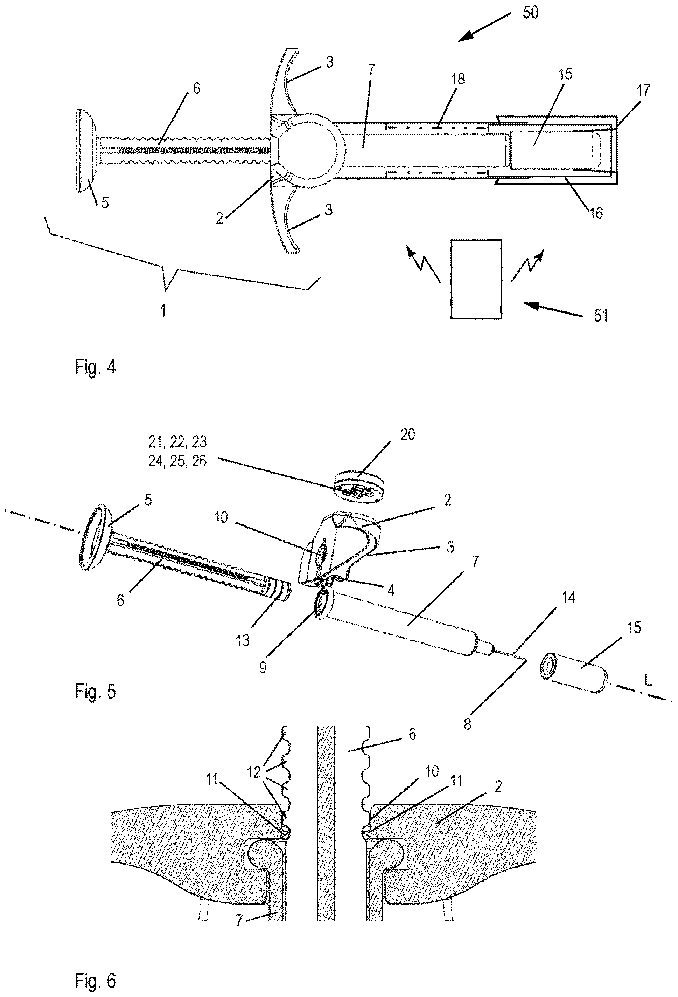

Figure 4 depicts a lateral view of a second embodiment of an operating assembly 1 and of an injection device 50 respectively according to the invention. The operating assembly 1 includes a fixture 2, a plunger rod 6 and a button 5. The injection device 50 includes said operating assembly 1 where a syringe 7 is fastened in the fixture 2. The fixture 2 forms a finger rest 3 on the distal side of a lateral or radially directed protrusion. The fixture 2 may operatively guide the plunger rod 6 for an axial movement e.g. an ejection stroke. At the proximal end of the plunger rod 6 a distinct button 5 is formed or attached.

The distal end of the syringe 7 is covered by a needle shield 15. A cap 17 is operatively coupled e.g. by gripping fingers or by a gripping sleeve with the needle shield 15 in order to remove the needle shield from the syringe 7 if the cap 17 is pulled of the injection device 50. A safety sleeve 16 is biased in distal direction by a safety spring 18 in order to expose the needle 14 if the distal end of the injection device 50 is pushed against an injection site and in order to cover the needle 14 if the injection device 50 is removed from the injection site. The cap 17 is removably coupled with the fixture 2 or with the syringe 7 by a housing sleeve which coaxially surrounds the barrel of the syringe 7. The safety spring 18 and the safety sleeve 16 are moveably accommodated in the annular gap between the barrel of the syringe 7 and the housing sleeve.

Also shown on figure 4 is a gateway device 51 e.g. a smartphone and/or a smartwatch deploying a software or app enabling data communication to the injection device 50 as well as to a remote host system.Figure 5 depicts an exploded perspective view of the injection device 50 respectively from figure 4. From left to right along the central axis L the following parts are shown: The button 5, the plunger rod 6, a stopper 13, the fixture 2, a data acquisition unit 20 accommodated in a laterally formed encasing of the fixture 2 includes a processor 21, a memory 22, a transceiver 23, a sensor 24, a battery 25 and a display 26. The display 26 e.g. a LED may be visible through a transparent sidewall of the fixture 2 or through the lateral encasing. A syringe 7 with a staked needle 14. The prefilled syringe 7 features a proximal filling end 9 and a distal needle end 8. The fixture 2 with the finger rest 3 features a lateral opening or clamping groove 4 and a central or coaxial opening or bearing 10. The bearing 10 is adapted to guide the plunger rod axially along the central axis L. The clamping groove 4 is adapted to take in and clamp the flange and a section of the barrel of the syringe 7. The releasable needle shield 15 is adapted to sealingly cover the staked needle 14.

Figure 6 depicts a sectional view of an axial section of the operating assembly 1 and the injection device 50 respectively from figure 4. The plunger rod 6 and the fixture 2 with the clamped syringe 7 is shown. The bearing 10 exhibits interaction structures 11,12 where a first interaction structure 11 may be formed by the fixture 2 by a radially inwardly directed protrusion and a second interaction structure 12 may be formed by the plunger rod 6 as radially outwardly directed and axially lined up protrusions. As the plunger rod 6 is guided and axially moved in the bearing 10 of the fixture 2 variable frictional force and/or plastic deformation occurs between and on the interaction structures 11, 12. Such acoustical and/or structure-borne sound is evoked and the speed of the plunger rod 6 is modulated periodically by the variation of the occurring frictional force as the plunger rod with its interaction structure 12 is moved by an external thrust in e.g. an injection or ejection stroke.

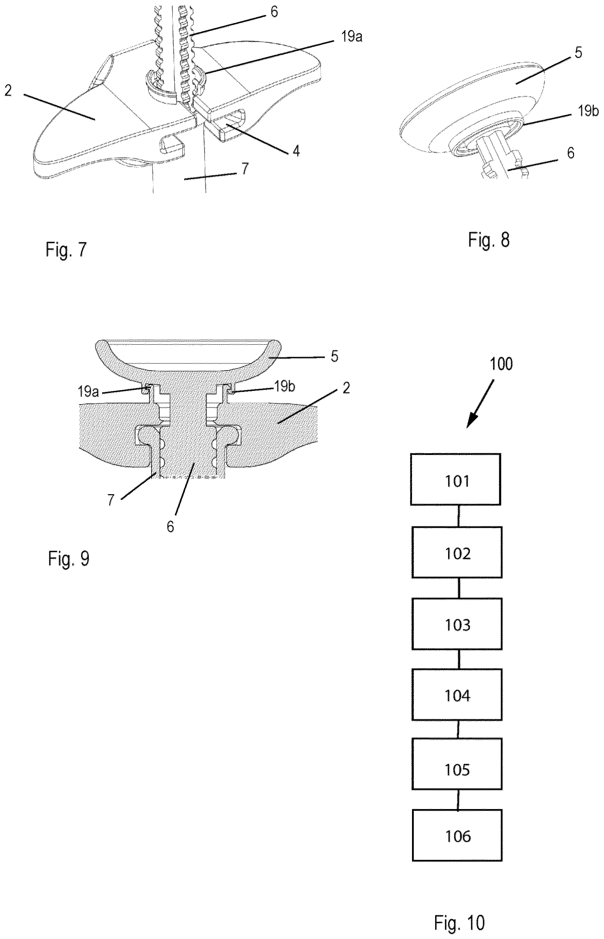

Figure 7 depicts a perspective view of an axial section of the operating assembly 1 and the injection device 50 respectively from figure 1 or figure 4. The fixture 2 guides the plunger rod 6 and clamps the prefilled syringe inserted in the clamping groove 4. Interlocking hooks 19a are directed proximally and formed as segments of a coaxial circular arc on the proximal side of the fixture 2. The proximal end of the interlocking hooks 19a protrudes radially outwards.

Figure 8 depicts a perspective view of the plunger rod 6 with the button 5 from figure 7. Interlocking hooks 19b are directed distally and formed as segments of a coaxial circular arc on the distal end plate of the button 5. The proximal end of the interlocking hooks 19b protrudes radially inwards.

Both interlocking hooks 19a and 19b are configured to form a non-releasable, positive fit connection once snapped in.

At the end of the ejection stroke the interlocking hooks 19a and 19b snap in irreversibly due to the ongoing and correspondingly enhanced thrust driving the plunger rod 6 into the syringe 7.

Figure 9 depicts a sectional view of an axial section of the operating assembly 1 and the injection device 50 respectively from figure 7 and figure 8 with snapped in interlocking hooks 19a, 19b where the plunger rod 6 is irreversibly locked in its distal end position to the fixture 2.

Figure 10 depicts a flow chart of the computer implemented method 100 carried out by the data acquisition unit 20 of the operating assembly 1, the injection device 50 or the system including the gateway device 51 or a remote host respectively from figure 1 or figure 4.

The computer implemented method 100 including the steps of:
- predefining 101, in the memory 22, predefined data patterns 30 characteristic for or relating to signals 27 evoked by at least one of acceleration, acoustical or structure borne sound or strain taking place in one or more of the plunger rod 6 or the fixture 2 or other parts e.g. the syringe 7 during intended use of the administration device 50
- capturing 102, by the sensor 24, the signal 27 evoked by at least one of acceleration, acoustical or structure borne sound or strain taking place with or inside one or more of the plunger rod 6, the fixture 2 or further involved parts or usage actions
- receiving 103, by the processor 21, from the sensor 24 the electrical representation of the signal 27 rendering in the time domain at least one of said acceleration, acoustical or structure borne sound or strain
- storing 104, in the memory 22, the digital representation of said signal 27 e.g. in the time domain
- transforming 105 and storing 104, by the processor 21, in the memory 22, the digital representation of the signal 27 into other domains, for example the frequency domain
- obtaining 106, by the processor 21, by comparing or correlating the digital representation of signal 27 and the predefined data patterns 30 in memory 22, metrics 28 assigned to the relative movement of the plunger rod 6 or the button 5 and the finger rest 3 or the fixture 2, for example a relative or absolute position and/or the speed of the plunger rod 6 with respect to to the other parts e.g. the fixture 2, the prefilled syringe 7 or other parts.

The method 100 may further include the steps of:
- converting, by the processor 21, the metrics 28 into an amount of medication expelled
- obtaining, by the processor 21, by comparing or correlating the digital representation of the signal 27 and the predefined patterns 30 in memory 22, device status information 29
- pairing, by the processor 21 and the transceiver 23, the data acquisition unit 20 with a remote gateway device 51, for example a smartphone
- transmitting, by the transceiver 23, metrics 28 and status information 29 to the remote gateway device 51
- receiving, by the transceiver 23, data to be stored in memory 22 or to be shown on the display 26

By way of example the processor 21, memory 22 and transceiver 23 implementing the method 100 may be a Flash ROM based system on chip (SoC) integrating e.g. an ARM Cortex Architecture and RAM. The SoC supporting Bluetooth Low Energy or NFC or Thread or Zigbee or LoRa or Mesh protocols as well as over-the-air device firmware upgrade capabilities.

While the invention has been described in detail in the drawings and foregoing description, such description is to be considered illustrative or exemplary and not restrictive. Variations to the disclosed embodiments can be understood and effected by those skilled in the art and practicing the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

| | | | |
|---|---|---|---|
| 1 | operating assembly | 50 | injection device |
| 2 | fixture | | |
| 3 | finger rest | 51 | gateway device |
| 4 | clamping groove | | |
| 5 | button | | |
| 5a | lid | | |
| 6 | plunger rod | 100 | method |
| 7 | prefilled syringe | 101 | predefining |
| 8 | distal needle end | 102 | capturing |
| 9 | proximal filling end | 103 | receiving |
| 10 | bearing | 104 | storing |
| 11 | first interaction structure (fixture) | 105 | transforming |
| 12 | second interaction structure (plunger rod) | 106 | obtaining |
| 13 | stopper | 107 | converting |
| 14 | staked needle | 108 | obtaining |
| 15 | needle shield | 109 | pairing |
| 16 | safety sleeve | 110 | transmitting |
| 17 | cap | 111 | receiving |
| 18 | safety spring | | |
| 19a | interlocking hooks | | |
| 19b | interlocking hooks | | |
| 20 | data acquisition unit | | |
| 21 | processor | L | central axis |
| 22 | memory | | |
| 23 | transceiver | | |
| 24 | sensor | | |
| 25 | battery | | |
| 26 | display | | |
| 27 | signal | | |
| 28 | metrics | | |
| 29 | status information | | |
| 30 | predefined data pattern | | |

## Claims

1. An operating assembly (1) for a prefilled syringe (7) comprising:
- a fixture (2) to accommodate the prefilled syringe (7), the prefilled syringe (7) defining a central axis running through its distal needle end (8) and its proximal filling end (9)
- a finger rest (3) couplable to or integrally formed by the fixture (2)
- a bearing (10) running with the central axis (L) provided by the fixture (2)
- a plunger rod (6) operatively coupled to the fixture (2) and axially movable in the bearing (10) relative to the fixture (2) in an ejection stroke from a proximal initial position to a distal end position, the plunger rod (6) comprising a button (5)
- a data acquisition unit (20) accommodated either by the fixture (2) or the plunger rod (6), the data acquisition unit (20) comprising: a processor (21), a memory (22), a transceiver (23), a sensor (24) and a battery (25), the sensor (24) comprising at least one of an acceleration detector, a microphone or a strain gauge
- at least two interaction structures (11, 12), whereby one of the interaction structures is provided by the fixture (2) and the other by the plunger rod (6),
wherein the interaction structures (11, 12) are mutually shaped and arranged in such a way a frictional force may act between them which occurs and /or varies repeatedly during an ejection stroke wherein the acceleration and the speed of both the fixture (2) and the plunger rod (6) are modulated accordingly,
wherein the data acquisition unit (20) captures a mechanical and/or acoustical signal (27) evoked by said modulated speed and acceleration.

2. The operating assembly (1) according to claim 1, wherein the interaction structure (12) provided by the plunger rod (6) comprises a plurality of radially directed protrusions and/or indentations formed along a surface section of the plunger rod (6) in an axial direction.

3. The operating assembly (1) according to any of claim 1 to 2, wherein the interaction structure (11) provided by the fixture (2) comprises at least one of a protrusion provided by the bearing (10)

4. The operating assembly (1) according to any of claim 1 to 3, wherein during the ejection stroke a mutual deformation and/or deflection of the interaction structures (11, 12) takes place

5. The operating assembly (1) according to any of claim 1 to 4, wherein the plunger rod (6) is moved axially through the bearing (10) and into the prefilled syringe (7) by a manual thrust acting on a proximal end plate of the button (5)

6. The operating assembly (1) according to any of claim 1 to 5, wherein the distal end plate of the button (5) and the proximal side of the fixture (2) are provided with interlocking hooks (19a, 19b)

7. The operating assembly (1) according to any of claim 1 to 6, wherein the sensor (24) of the data acquisition unit (20) further comprises a pressure detector capable of capturing a force acting on the button (5) from the outside

8. The operating assembly (1) according to any of claim 1 to 7, wherein the data acquisition unit (20) further comprises a display (26), for example a LED providing status information

9. An injection device (50) comprising an operating assembly (1) according to the claims 1 to 8, the injection device (50) further comprising:
- a prefilled syringe (7) having a stopper (13) and a staked needle (14), the staked needle (14) covered by a removable needle shield (15), the prefilled syringe (7) accommodated in the operating assembly (1)

10. The injection device (50) according to claim 9, the injection device (50) further comprising:
- a safety sleeve (16) telescopically movable with respect to the prefilled syringe (7) capable to expose the needle (14) for injection

11. The injection device (50) according to the claims 9 or 10, the injection device (50) further comprising:
- a cap (17), operatively coupled to the needle shield (15) and adapted to remove the needle shield (15) from the prefilled syringe (7) together with the cap (17)

12. A computer-implemented method (100) carried out by the data acquisition unit (20) of the operating assembly (1) or the injection device (50) respectively according to claims 1 to 11, the method (100) comprising the steps of:
- predefining (101), in the memory (22), data patterns (30) characteristic for or relating to the signal (27) evocable by at least one of acceleration, structure borne sound or strain taking place with or inside one or more of the plunger rod (6) or the fixture (2) during intended use of the administration device (50)
- capturing (102), by the sensor (24), signal (27) evoked by at least one of acceleration, structure borne sound or strain taking place in one or more of the plunger rod (6) or the fixture (2)
- receiving (103), by the processor (21), from the sensor (24) an electrical representation of the signal (27) representing in the time domain at least one of said acceleration, structure borne sound or strain
- storing (104), in the memory (22), the digital representation of said signal (27) e.g. in the time domain
- transforming (105) and storing (104), by the processor (21), in memory (22), the digital representation of the signal (27) into other domains, for example the frequency domain
- obtaining (106), by the processor (21), by comparing or correlating the digital representation of the signal (27) and the predefined patterns in memory (22), metrics (28) assigned to the relative movement of the plunger rod (6) and the fixture (2), for example a relative or absolute position and/or the speed of the plunger rod (6) with respect to the other parts (2,3,7).

13. The method (100) according to claim 12, the method (100) further comprising at least one of the steps of:
- converting (107), by the processor (21), the metrics (28) into an amount of medication expelled
- obtaining (108), by the processor (21), by comparing or correlating the digital representation of the signal (27) and the predefined patterns (30) in memory (22), device status information (29)

14. The method (100) according to claim 12 or 13, the method (100) further comprising the steps of:
- pairing (109), by the processor (21) and the transceiver (23), the data acquisition unit (20) with a remote gateway device (51), for example a smartphone
- transmitting (110), by the transceiver (23), metrics (28) and status information (29) to the remote gateway device (51)
- receiving (111), by the transceiver (23), data to be stored in memory (22) or to be shown on the display (26)

15. The method according to claim 12 to 14, whereby the predefined data patterns (30) are obtained by a training session of a neuronal network or by machine learning or the use of artificial intelligence.
